# EUROPEAN PATENT APPLICATION

(11) **EP 4 385 505 A1**
(43) Date of publication of application: **19.06.2024**
(21) Application number: 22894599.4
(22) Date of filing: 27.10.2022
(51) Int. Cl.: A61K 31/136, A61K 31/167, A61K 31/223, A61K 31/27, A61P 43/00

(54) **ANTI-HYPOXIC/ANOXIC INJURY USE OF MAGNOLOL AND/OR HONOKIOL AROMATIC RING AMINO-SUBSTITUTED DERIVATIVE, AND PHARMACEUTICAL COMPOSITION**

(30) Priority: 19.11.2021 CN 202111402114
(71) Applicant: Beijing Honghui Meditech Co., Ltd., Beijing 102600 (CN)
(72) Inventor: ZHANG, Pingping, Beijing 102600 (CN); LIU, Ye, Beijing 102600 (CN); ZHANG, Yuying, Beijing 102600 (CN)
(74) Representative: HGF
(86) International application number: PCT/CN2022/128028
(87) International publication number: WO 2023/088062

(57) **Abstract**

The present disclosure provides an anti-hypoxic/anoxic injury use of a magnolol and/or honokiol aromatic ring amino-substituted derivative, and a pharmaceutical composition. The magnolol and/or honokiol aromatic ring amino-substituted derivative is a compound represented by general formula I or a salt thereof: 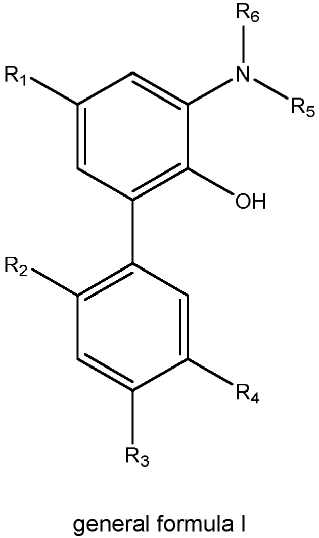 in general formula I, R₁ and R₄ are each independently selected from a C₁-C₈ hydrocarbyl, R₂ and R₃ are each independently selected from hydrogen or hydroxyl, and R₂ and R₃ are not both the hydrogen or hydroxyl; R₅ is selected from any one of H, a C₁-C₁₂ acyl, a remaining acyl moiety of a single amino acid after condensation of its carboxyl, or a remaining acyl moiety of a polypeptide after condensation of its carboxyl; and R₆ is selected from any one of hydrogen or C₁-C₈ hydrocarbyl. The derivative of the structure shown in general formula I may be used for preparing an anti-hypoxic/anoxic injury drug, provides a new drug option for prevention and treatment of diseases, and solves problems of indirect onset and slow onset and the like of existing anti-hypoxic/anoxic drugs.

## Description

### Cross-Reference to Related Application

The present application is based on and claims priority to Chinese application with CN application number of 202111402114.5 filed on November 19, 2021, the disclosure of which is hereby incorporated again by reference in its entirety.

### Technical Field

The present disclosure relates to the technical field of biopharmaceuticals, in particular to an anti-hypoxic/anoxic injury use of a magnolol and/or honokiol aromatic ring amino-substituted derivative, and a pharmaceutical composition.

### Background

Magnolol and honokiol are main active ingredients of traditional Chinese medicine cortex magnoliae officinalis, and are usually obtained by structural modifications of traditional Chinese medicine cortex magnoliae officinalis extracts. The chemical structural formulas of the magnolol and honokiol are respectively as follows:

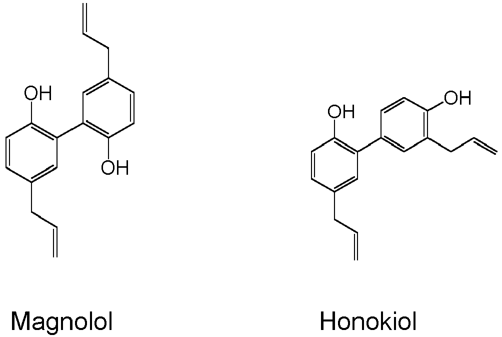

In 1930, Sugii in Japan firstly separates the magnolol from Chinese magnoliae officinalis barks (Chinese herbal medicine: 2005, 36, 10, 1591-1594). In 1989, Meng Lizhen, et al. in China also separates the honokiol from magnoliae officinalis (Chinese patent medicine: 1989, 11 (8): 223). The magnolol and honokiol have a wide range of pharmacological effects, such as anti-bacterial, anti-inflammatory, anti-tumor, muscle relaxation, cholesterol lowering, and anti-aging (Chinese herbal medicine: 2005, 36, 10, 1591-1594). Drugs used in this article are magnolol and honokiol aromatic ring amino-substituted derivatives, and there are currently no reports on uses to resist anti-hypoxic/anoxic injuries.

High altitude hypoxia (HAH) poses a potential threat to the health of humans living in this region for a long time. The heart is one of organs with the relatively high oxygen consumption in the body, and maintaining the vitality and function of the heart requires sufficient oxygen supply. Exposure to a low-pressure hypoxic environment, due to the decrease in oxygen partial pressure, reduces the supply of oxygen to the heart, and cardiac dysfunction is caused, thus the occurrence of some cardiovascular diseases is triggered, such as myocardial infarction (MI) and right ventricular (RV) dysfunction (induced by pulmonary arterial hypertension), and even sudden cardiac death (SCD). The brain is another organ in the body with the relatively high oxygen consumption and extremely low tolerance to hypoxia, the low-pressure hypoxic environment may cause irreversible injuries to the brain. In recent years, with the increasing number of people rushing to plateaus, the people living in plains have rapidly decreased atmospheric pressure and oxygen partial pressure after rushing to the plateaus, the body's blood oxygen saturation is also rapidly decreased, and tissues are quickly located in a hypoxic state, thereby a series of physiological and pathological changes in the body is caused, the body may experience acute mountain sickness, palpitations, chest tightness, chest pain, dizziness, breathing difficulties and the like, and severe cases may even lead to acute pulmonary edema and other conditions, and even endanger the life.

At present, traditional Chinese medicines in anti-hypoxic drugs include Rhodiola, Angelica sinensis, Ginseng and the like, and it has a relatively definite therapeutic effect, but takes effect slowly; and western medicines include acetazolamide, dexamethasone, aminophylline tablets and the like, although these drugs have a relieving effect on hypoxia symptoms, it may not directly increase the blood oxygen saturation in the body so as to prevent and resist the injuries caused by the hypoxic/anoxic environment.

### Summary

A main purpose of the present disclosure is to provide an anti-hypoxic/anoxic injury use of a magnolol and/or honokiol aromatic ring amino-substituted derivative, and a pharmaceutical composition, as to solve problems of indirect onset and slow onset of existing anti-hypoxic drugs.

In order to achieve the above purpose, according to one aspect of the present disclosure, an anti-hypoxic/anoxic injury use of a magnolol and/or honokiol aromatic ring amino-substituted derivative is provided, and the magnolol and/or honokiol aromatic ring amino-substituted derivative is a compound shown in general formula I or a salt thereof:

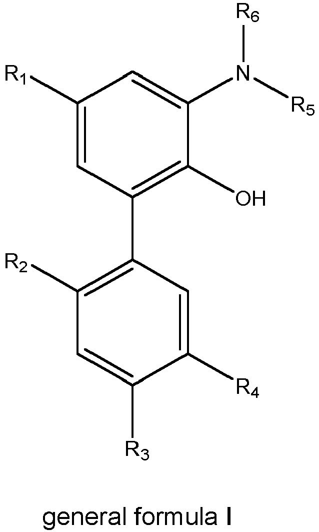

In general formula I, R₁ and R₄ are each independently selected from a C₁-C₈ hydrocarbyl, R₂ and R₃ are each independently selected from hydrogen or hydroxyl, and R₂ and R₃ are not both the hydrogen or hydroxyl; R₅ is selected from any one of H, a C₁-C₁₂ acyl, a remaining acyl moiety of a single amino acid after condensation of its carboxyl, or a remaining acyl moiety of a polypeptide after condensation of its carboxyl; and R₆ is selected from any one of hydrogen or C₁-C₈ hydrocarbyl.

Further, the C₁-C₈ hydrocarbyls in the above R₁, R₄, and R₆ are each independently selected from any one of a C₁-C₈ alkyl or a C₁-C₈ alkenyl, and preferably the C₁-C₈ alkyl is selected from any one of methyl, ethyl, propyl, butyl, pentyl, hexyl, heptyl, or octyl.

Further, the above C₁-C₈ alkenyl is selected from any one of ethenyl, propenyl, allyl, butan-1-enyl, butan-2-enyl, butan-3-enyl, pentan-1-enyl, pentan-2-enyl, pentan-3-enyl, pentan-4-enyl, hex-1-enyl, hex-2-enyl, hex-3-enyl, hex-4-enyl, hex-5-enyl, hept-1-enyl, hept-2-enyl, hept-3-enyl, hept-4-enyl, hept-5-enyl, hept-6-enyl, oct-1-enyl, oct-2-enyl, oct-3-enyl, oct-4-enyl, oct-5-enyl, oct-6-enyl, or oct-7-enyl.

Further, the above C₁-C₁₂ acyl is selected from any one of formyl, acetyl, propionyl, butyryl, valeryl, hexanoyl, heptanoyl, or octanoyl.

Further, the above single amino acid is selected from any one of lysine, methionine, tryptophan, valine, alanine, phenylalanine, leucine, isoleucine, 6-hydroxyleucine, glycine, histidine, arginine, proline, glutamic acid, aspartic acid, serine, threonine, tyrosine, cystine or cysteine.

Further, the above polypeptide is a peptide formed by a plurality of single amino acids, and preferably the molecular weight of the polypeptide is ≤2500 Da.

Further, when the above magnolol and/or honokiol aromatic ring amino-substituted derivative is the salt of the compound shown in general formula I, one or more of salifiable amino groups carried by R₅ are in salt form, and an acid used for salt formation is a pharmaceutically acceptable acid such as hydrochloric acid, oxalic acid, or fumaric acid.

Further, the above magnolol and/or honokiol aromatic ring amino-substituted derivative is any one or more of the following compounds: 3',5-diallyl-3-amino-[1,1'-biphenyl]-2,4'-diol and hydrochloride thereof, N-(3',5-diallyl-2,4'-dihydroxy-[1,1'-biphenyl]-3-yl)acetamide, (S)-2,6-diamino-N-(3',5-diallyl-2,4'-dihydroxy-[1,1'-biphenyl]-3-yl)hexanamide and hydrochloride thereof, (S)-2-amino-N-(3',5-diallyl-2,4'-dihydroxy-[1,1'-biphenyl]-3-yl)-3-phenylpropanamide and hydrochloride thereof, (R)-2,6-diamino-N-(3',5-diallyl-2,4'-dihydroxy-[1,1'-biphenyl]-3-yl)hexanamide and hydrochloride thereof, (S)-2-amino-N-(3',5-diallyl-2,4'-dihydroxy-[1,1'-biphenyl]-3-yl)-6-hydroxyhexanamide and hydrochloride thereof, (S)-2-amino-N-(3',5-diallyl-2,4'-dihydroxy-[1,1'-biphenyl]-3-yl)-4-(methylthio)butanamide and hydrochloride thereof, (S)-2-amino-N-(3',5-diallyl-2,4'-dihydroxy-[1,1'-biphenyl]-3-yl)-3-methylbutanamide and hydrochloride thereof, and (S)-2,6-diamino-N-(2,4'-dihydroxy-3',5-dipropyl-[1,1'-biphenyl]-3-yl)hexanamide and hydrochloride thereof.

Further, the administration mode of the above anti-hypoxic/anoxic injury drug is injection, oral administration, implantation, or direct filling of lesion sites.

According to another aspect of the present disclosure, an anti-hypoxic/anoxic injury pharmaceutical composition is provided, and the anti-hypoxic/anoxic injury pharmaceutical composition includes a magnolol and/or honokiol aromatic ring amino-substituted derivative or a salt thereof, as well as a pharmaceutically acceptable carrier; and herein the magnolol and/or honokiol aromatic ring amino-substituted derivative is the above magnolol and/or honokiol aromatic ring amino-substituted derivative.

By applying technical schemes of the present disclosure, the derivative with the structure shown in the above general formula I may be used to prepare the anti-hypoxic/anoxic injury drug, rapidly and effectively improve the oxygen carrying capacity of red blood cells, provide a new drug choice for prevention and treatment of diseases (such as altitude sickness) caused by external hypoxia and breathing and/or ventilation dysfunction diseases caused by various self-causes (such as central nervous system diseases, bronchial and pulmonary lesions), and solve problems of indirect onset and slow onset and the like of existing anti-hypoxic/anoxic drugs.

### Brief Description of the Drawings

Drawings of the description constituting a part of the present disclosure are used to provide further understanding of the present disclosure. Schematic embodiments of the present disclosure and descriptions thereof are used to explain the present disclosure, and do not constitute improper limitations on the present disclosure. In the drawings:
Fig. 1 shows an effect diagram of Compound 1 on blood oxygen partial pressure (PO₂) and blood oxygen saturation (SO₂) of Sprague Dawley (SD) rats before and after 24 h of hypoxia treatment according to Embodiment 1 of the present disclosure;
Fig. 2 shows an effect diagram of Compound 1 on the respiratory rate (RR) of the SD rats before and after 24 h of the hypoxia treatment according to Embodiment 1 of the present disclosure;
Fig. 3 shows an effect diagram of Compound 2 intravenously injected on rat blood oxygen partial pressure (PO₂) and blood oxygen saturation (SO₂) before 24 h of the hypoxia treatment and after 1 h, 6 h, and 24 h of the hypoxia treatment according to Embodiment 2 of the present disclosure;
Fig.4 shows an effect diagram of Compound 2 intravenously injected on the RR of the SD rats before 24 h of the hypoxia treatment and after 6 h and 24 h of the hypoxia treatment according to Embodiment 2 of the present disclosure;
Fig. 5 shows an effect diagram of Compound 2 intravenously injected on lung tissue injuries of the SD rats after 24 h of the hypoxia treatment according to Embodiment 2 of the present disclosure;
Fig. 6 shows an effect diagram of Compound 2 intragastrically administered on the blood oxygen partial pressure (PO₂) and blood oxygen saturation (SO₂) before and after 24 h of the hypoxia treatment according to Embodiment 3 of the present disclosure;
Fig. 7 shows an effect diagram of Compound 2 intragastrically administered on the rat RR before and after 24 h of the hypoxia treatment according to Embodiment 3 of the present disclosure;
Fig. 8 shows an effect diagram of Compound 3 on the blood oxygen saturation (SO₂) of the SD rats before and after 24 h of the hypoxia treatment according to Embodiment 4 of the present disclosure;
Fig. 9 shows an effect diagram of Compound 3 on the rat RR before and after 24 h of the hypoxia treatment according to Embodiment 4 of the present disclosure;
Fig. 10 shows an effect diagram of Compound 4 on the blood oxygen partial pressure (PO₂) and blood oxygen saturation (SO₂) of the SD rats before and after 24 h of the hypoxia treatment according to Embodiment 5 of the present disclosure; and
Fig. 11 shows an effect diagram of Compound 4 on the rat RR before and after 24 h of the hypoxia treatment according to Embodiment 5 of the present disclosure;

### Detailed Description of the Embodiments

It should be noted that embodiments in the present disclosure and features in the embodiments may be combined with each other in the case without conflicting. The present disclosure is described in detail below with reference to drawings and in combination with the embodiments.

As analyzed by the background, existing anti-hypoxic drugs have a problem of indirect onset and slow onset. In order to solve this problem, the present disclosure provides an anti-hypoxic/anoxic injury use of a magnolol and/or honokiol aromatic ring amino-substituted derivative, and a pharmaceutical composition.

In a typical implementation mode of the present disclosure, an anti-hypoxic/anoxic injury use of a magnolol and/or honokiol aromatic ring amino-substituted derivative is provided, and the magnolol and/or honokiol aromatic ring amino-substituted derivative is a compound shown in general formula I or a salt thereof:

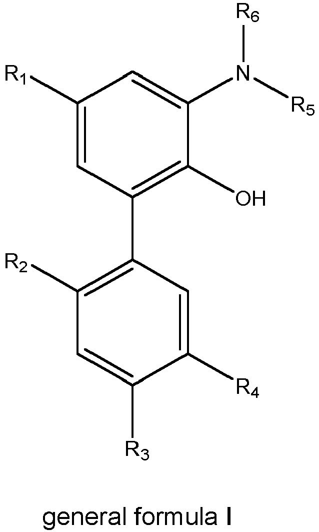

In general formula I, R₁ and R₄ are each independently selected from a C₁-C₈ hydrocarbyl, R₂ and R₃ are each independently selected from hydrogen or hydroxyl, and R₂ and R₃ are not both the hydrogen or hydroxyl; R₅ is selected from any one of H, a C₁-C₁₂ acyl, a remaining acyl moiety of a single amino acid after condensation of its carboxyl, or a remaining acyl moiety of a polypeptide after condensation of its carboxyl; and R₆ is selected from any one of hydrogen or C₁-C₈ hydrocarbyl.

The derivative with the structure shown in the above general formula I may be used to prepare the anti-hypoxic/anoxic injury drug, rapidly and effectively improve the oxygen carrying capacity of red blood cells, provide a new drug choice for prevention and treatment of diseases (such as altitude sickness) caused by external hypoxia and breathing and/or ventilation dysfunction diseases caused by various self-causes (such as central nervous system diseases, bronchial and pulmonary lesions), and solve problems of indirect onset and slow onset and the like of the existing anti-hypoxic/anoxic drugs.

In order to further improve the synthesis efficiency of the magnolol and/or honokiol aromatic ring amino-substituted derivative, and further explore the use thereof in preparation of the anti-hypoxic/anoxic injury drug, preferably the C₁-C₈ hydrocarbyls in the above R₁, R₄, and R₆ are each independently selected from any one of a C₁-C₈ alkyl or a C₁-C₈ alkenyl, and preferably the C₁-C₈ alkyl is selected from any one of methyl, ethyl, propyl, butyl, pentyl, hexyl, heptyl, or octyl, and further preferably the C₁-C₈ alkyl is selected from the propyl.

R₁ and R₄ are respectively located at a para or ortho position of a hydroxyl in each benzene ring, and its electronic effect and spatial effect may affect the reaction activity of each site on the benzene ring. In order to make the sites such as the hydroxyl and the hydroxyl ortho position on each benzene ring have the relatively high reaction activity as high as possible, as to obtain a drug with an excellent anti-hypoxic/anoxic property more easily, preferably the above C₁-C₈ alkenyl is selected from any one of ethenyl, propenyl, allyl, butan-1-enyl, butan-2-enyl, butan-3-enyl, pentan-1-enyl, pentan-2-enyl, pentan-3-enyl, pentan-4-enyl, hex-1-enyl, hex-2-enyl, hex-3-enyl, hex-4-enyl, hex-5-enyl, hept-1-enyl, hept-2-enyl, hept-3-enyl, hept-4-enyl, hept-5-enyl, hept-6-enyl, oct-1-enyl, oct-2-enyl, oct-3-enyl, oct-4-enyl, oct-5-enyl, oct-6-enyl, or oct-7-enyl.

In order to further improve the synthesis convenience of the magnolol and/or honokiol aromatic ring amino-substituted derivative, preferably the above R₁ and R₄ are independently the allyl respectively.

Preferably the above C₁-C₁₂ acyl is selected from any one of formyl, acetyl, propionyl, butyryl, valeryl, hexanoyl, heptanoyl, or octanoyl. The above acyl is relatively easy to obtain and is more likely to exert its anti-hypoxic/anoxic activity. Further, the C₁-C₁₂ acyl is preferably the acetyl.

In one embodiment of the present disclosure, the above single amino acid is selected from any one of lysine, methionine, tryptophan, valine, alanine, phenylalanine, leucine, isoleucine, 6-hydroxyleucine, glycine, histidine, arginine, proline, glutamic acid, aspartic acid, serine, threonine, tyrosine, cystine or cysteine.

The above types of the amino acids are relatively easy to obtain, and most of the amino acids are required by the human body, which is more economical and safe. In order to further improve the beneficial effects of the above magnolol and/or honokiol aromatic ring amino-substituted derivative on the human body anti-hypoxic/anoxic activity, preferably the above amino acid is the lysine or the methionine.

In order to further improve the pharmacological activity of R₅ as a polypeptide, preferably the above polypeptide is a peptide formed by a plurality of the above single amino acids, and preferably the molecular weight of the polypeptide is ≤2500 Da.

In one embodiment of the present disclosure, when the above magnolol and/or honokiol aromatic ring amino-substituted derivative is the salt of the compound shown in general formula I, one or more of salifiable amino groups carried by R₅ are in salt form, and an acid used for salt formation is a pharmaceutically acceptable acid such as hydrochloric acid, oxalic acid, or fumaric acid.

When the above magnolol and/or honokiol aromatic ring amino-substituted derivative is the pharmaceutically acceptable salt of the compound shown in general formula I, its water solubility is good, and the application is more convenient.

In some embodiments, preferably the above magnolol and/or honokiol aromatic ring amino-substituted derivative is any one or more of the following compounds:
3',5-diallyl-3-amino-[1,1'-biphenyl]-2,4'-diol and hydrochloride thereof, N-(3',5-diallyl-2,4'-dihydroxy-[1,1'-biphenyl]-3-yl)acetamide,
(S)-2,6-diamino-N-(3',5-diallyl-2,4'-dihydroxy-[1,1'-biphenyl]-3-yl)hexanamide and hydrochloride thereof,
(S)-2-amino-N-(3',5-diallyl-2,4'-dihydroxy-[1,1'-biphenyl]-3-yl)-3-phenylpropanamide and
hydrochloride thereof, (R)-2,6-diamino-N-(3',5-diallyl-2,4'-dihydroxy-[1,1'-biphenyl]-3-yl)hexanamide and
hydrochloride thereof, (S)-2-amino-N-(3',5-diallyl-2,4'-dihydroxy-[1,1'-biphenyl]-3-yl)-6-hydroxyhexanamide and
hydrochloride thereof, (S)-2-amino-N-(3',5-diallyl-2,4'-dihydroxy-[1,1'-biphenyl]-3-yl)-4-(methylthio)butanamide and hydrochloride thereof,
(S)-2-amino-N-(3',5-diallyl-2,4'-dihydroxy-[1,1'-biphenyl]-3-yl)-3-methylbutanamide and hydrochloride thereof, and
(S)-2,6-diamino-N-(2,4'-dihydroxy-3',5-dipropyl-[1,1'-biphenyl]-3-yl)hexanamide and hydrochloride thereof.

Specifically, when the magnolol and/or honokiol aromatic ring amino-substituted derivative is preferably 3',5-diallyl-3-amino-[1,1'-biphenyl]-2,4'-diol and hydrochloride thereof, N-(3',5-diallyl-2,4'-dihydroxy-[1,1'-biphenyl]-3-yl)acetamide, (S)-2,6-diamino-N-(3',5-diallyl-2,4'-dihydroxy-[1,1'-biphenyl]-3-yl)hexanamide and hydrochloride thereof, (S)-2-amino-N-(3',5-diallyl-2,4'-dihydroxy-[1,1'-biphenyl]-3-yl)-4-(methylthio)butanamide and hydrochloride thereof, and (S)-2,6-diamino-N-(2,4'-dihydroxy-3',5-dipropyl-[1,1'-biphenyl]-3-yl)hexanamide and hydrochloride thereof, it is more beneficial to improve the anti-hypoxic property of the magnolol and/or honokiol aromatic ring amino-substituted derivative.

In order to improve the practicality of the above magnolol and/or honokiol aromatic ring amino-substituted derivative, preferably the administration mode of the above anti-hypoxic/anoxic injury drug is injection, oral administration, implantation, or direct filling of lesion sites.

In another typical implementation mode of the present disclosure, an anti-hypoxic/anoxic injury pharmaceutical composition is provided, and the anti-hypoxic/anoxic injury pharmaceutical composition includes a magnolol and/or honokiol aromatic ring amino-substituted derivative or a salt thereof, as well as a pharmaceutically acceptable carrier; and herein the magnolol and/or honokiol aromatic ring amino-substituted derivative is the above magnolol and/or honokiol aromatic ring amino-substituted derivative shown in general formula I.

The derivative with the structure shown in the above general formula I may be used to prepare the anti-hypoxic/anoxic injury drug, rapidly and effectively improve the oxygen carrying capacity of red blood cells, provide a new drug choice for prevention and treatment of diseases (such as altitude sickness) caused by external hypoxia and breathing and/or ventilation dysfunction diseases caused by various self-causes (such as central nervous system diseases, bronchial and pulmonary lesions), and solve problems of indirect onset and slow onset and the like of the existing anti-hypoxic/anoxic drugs.

The beneficial effects of the present disclosure are described below in combination with specific embodiments and contrast examples.

The magnolol and/or honokiol aromatic ring amino-substituted derivative used in the following embodiments may be prepared by using known routes in existing technologies. For example, it may be prepared by using a route in a patent CN103113264A.

### Anti-hypoxic/anoxic injury experiment:

### Embodiment 1

### 3', 5-diallyl-3-amino-[1,1'-biphenyl]-2,4'-diol (Compound 1):

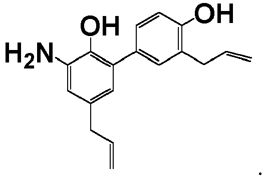

### I. Experimental method

1. Preparation of test substance: an appropriate amount of Compound 1 was weighed, and an appropriate amount of solvent 2% dimethylsulfoxide (DMSO) + 2% Tween80 + physiological saline was added, to prepare solution with a required concentration (0.58 mg/mL).
2. Animal model: SPF-grade SD male rats, 220-240 g of the weight, and hypoxia (the oxygen content was 11%) treatment.
3. Experimental grouping: a hypoxia treatment control group, there were 4 rats in each group; and a hypoxia treatment administration group, there were 4 rats in each group.
4. Administration mode: before the hypoxia treatment, a single intravenous injection was administered to each rat in the hypoxia treatment administration group.
5. Blood gas analysis: blood (approximately 0.5 mL) was respectively taken from femoral arteries of each group before and after 24 h of the hypoxia treatment for blood gas analysis, and it referred to Table 1 for details.

**Table 1**

| Group | Dose concentration (mg/kg) | Blood gas analysis time |
|---|---|---|
| Hypoxia treatment control group | - | Blood is taken once before 24 h of the hypoxia treatment; and blood is taken once after 24 h of the hypoxia treatment |
| Hypoxia treatment administration group | 0.58 | Blood is taken once before 24 h of the hypoxia treatment; and blood is taken once after 24 h of the hypoxia treatment |

6. Respiratory rate: the rat respiratory rate was evaluated during blood collection.
7. Data statistics: GraphPad was used for data analysis, and results were expressed as Mean ± Standard Deviation (Mean±SD). Single factor analysis of variance - T-test was used for data analysis, and P<0.05 represented that it had a statistical difference.

### II. Experimental result

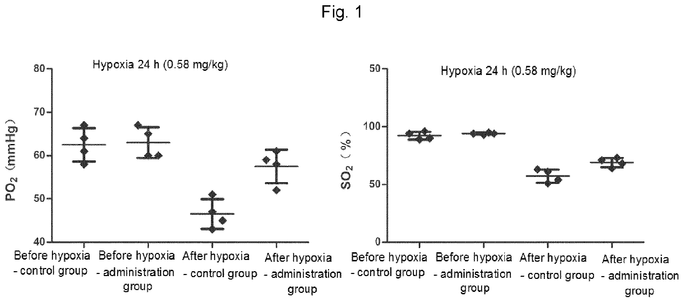

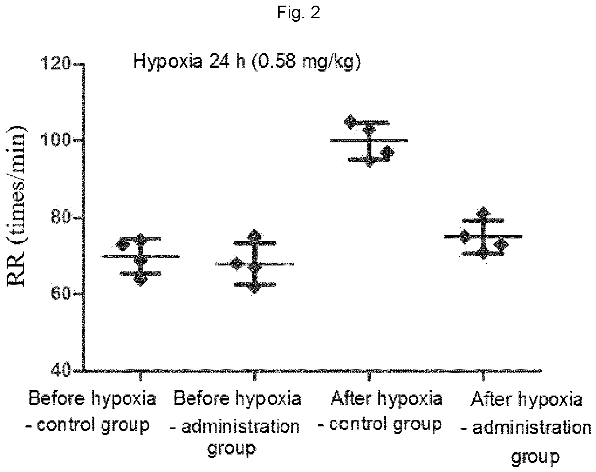

1. Blood gas analysis results were shown in Fig. 1 (namely the effects of Compound 1 on the blood oxygen partial pressure (PO₂) and blood oxygen saturation (SO₂) of the SD rats before and after 24 h of the hypoxia treatment):
   1) Before 24 h of the hypoxia treatment, there was no significant difference in the blood gas analysis results among each group of animals.
   2) After 24 h of the hypoxia treatment, the blood oxygen partial pressure (p=0.0012) and blood oxygen saturation (p=0.019) of the 0.58 mg/kg administration group were significantly higher than those of the control group.
2. The respiratory rate was shown in Fig. 2 (namely the effects of Compound 1 on the respiratory rate (RR) of the SD rats before and after 24 h of the hypoxia treatment):
   1) Before 24 h of the hypoxia treatment, there was no significant difference in the respiratory rate among each group of the animals.
   2) After 24 h of the hypoxia treatment, the respiratory rate of the administration group was lower than that of the control group, and the different was significant (p<0.05).

From the above data, it may be seen that 0.58 mg/kg of Compound 1 injected once may significantly increase the blood oxygen partial pressure and blood oxygen saturation of the SD rats after 24 h of the hypoxia treatment, significantly reduce the respiratory rate, and thus play an anti-hypoxic/anoxic injury role.

### Embodiment 2

### (S)-2,6-diamino-N-(3',5-diallyl-2,4'-dihydroxy-[1,1'-biphenyl]-3-yl)hexanamide dihydrochloride (Compound 2):

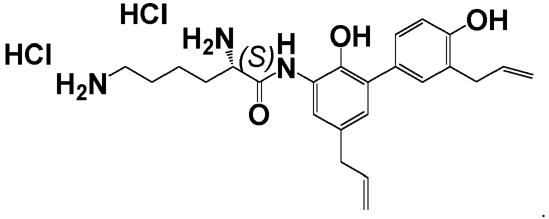

### I. Experimental method

1. Preparation of test substance: an appropriate amount of Compound 2 was weighed, and an appropriate amount of sodium chloride injection (0.9%) was added, to prepare solution with a required concentration (0.25 mg/kg and 1 mg/kg).
2. Animal model: SPF-grade SD male rats, 220-240 g of the weight, and hypoxia (the oxygen content was 11%) treatment.
3. Experimental grouping: a hypoxia treatment control group, there were 4 rats in each group; and a hypoxia treatment administration group, there were 4 rats in each group.
4. Administration mode: before the hypoxia treatment, a single intravenous injection was administered to each rat in the hypoxia treatment administration group.
5. Blood gas analysis: blood (approximately 0.5 mL) was respectively taken from femoral arteries of each group before and after 24 h of the hypoxia treatment for blood gas analysis, and it referred to Table 2 for details.

**Table 2**

| Group | Dose concentration (mg/kg) | Blood gas analysis time |
|---|---|---|
| Hypoxia treatment control group | - | Blood is taken once before 24 h of the hypoxia treatment; and blood is taken once after 1 h of the hypoxia treatment |
| Hypoxia treatment administration group | 0.25 | Blood is taken once before 24 h of the hypoxia treatment; and blood is taken once after 1 h of the hypoxia treatment |
| Hypoxia treatment control group | - | Blood is taken once before 24 h of the hypoxia treatment; and blood is taken once after 6 h of the hypoxia treatment |
| Hypoxia treatment administration group | 0.25 | Blood is taken once before 24 h of the hypoxia treatment; and blood is taken once after 6 h of the hypoxia treatment |
| Hypoxia treatment control group | - | Blood is taken once before 24 h of the hypoxia treatment; and blood is taken once after 6 h of the hypoxia treatment |
| Hypoxia treatment administration group | 1 | Blood is taken once before 24 h of the hypoxia treatment; and blood is taken once after 6 h of the hypoxia treatment |
| Hypoxia treatment control group | - | Blood is taken once before 24 h of the hypoxia treatment; and blood is taken once after 24 h of the hypoxia treatment |
| Hypoxia treatment administration group | 1 | Blood is taken once before 24 h of the hypoxia treatment; and blood is taken once after 24 h of the hypoxia treatment |

6. Respiratory rate: the rat respiratory rate was evaluated during blood collection.
7. Hematoxylin-eosin (H&E) staining:

Lung tissues of the hypoxia treatment (24 h) control group and the hypoxia treatment (24 h) administration group (dose: 1 mg/kg) were taken for paraffin section preparation, staining, and case analysis.
1) Paraffin sections were dewaxed to water: the sections were sequentially placed in xylene I for 20 min, xylene II for 20 min, anhydrous ethanol I for 5 min, anhydrous ethanol II for 5 min, and 75% alcohol for 5 min, and finally washed with tap water.
2) Hematoxylin staining: the sections were placed in hematoxylin staining solution and stained for 3-5 min, then washed with the tap water, and differentiated with differentiation solution, then washed with the tap water. It returned to blue by blue returning solution, and finally it was washed with running water.
3) Eosin staining: the sections were sequentially dehydrated with 85% and 95% of gradient alcohol for 5 min respectively, and placed in eosin staining solution and stained for 5 min.
4) Dehydration and section sealing: the sections were sequentially placed in anhydrous ethanol I for 5 min, anhydrous ethanol II for 5 min, anhydrous ethanol III for 5 min, xylene I for 5 min, and xylene II for 5 min until transparent, and finally sealed with a neutral resin.
5) Microscopic examination, image acquisition and analysis.
6) Result interpretation: the cell nucleus was blue and the cell cytoplasm was red.

8. Data statistics: GraphPad was used for data analysis, and results were expressed as Mean±SD. Single factor analysis of variance - T-test was used for data analysis, and P<0.05 represented that it had a statistical difference.

### II. Experimental result

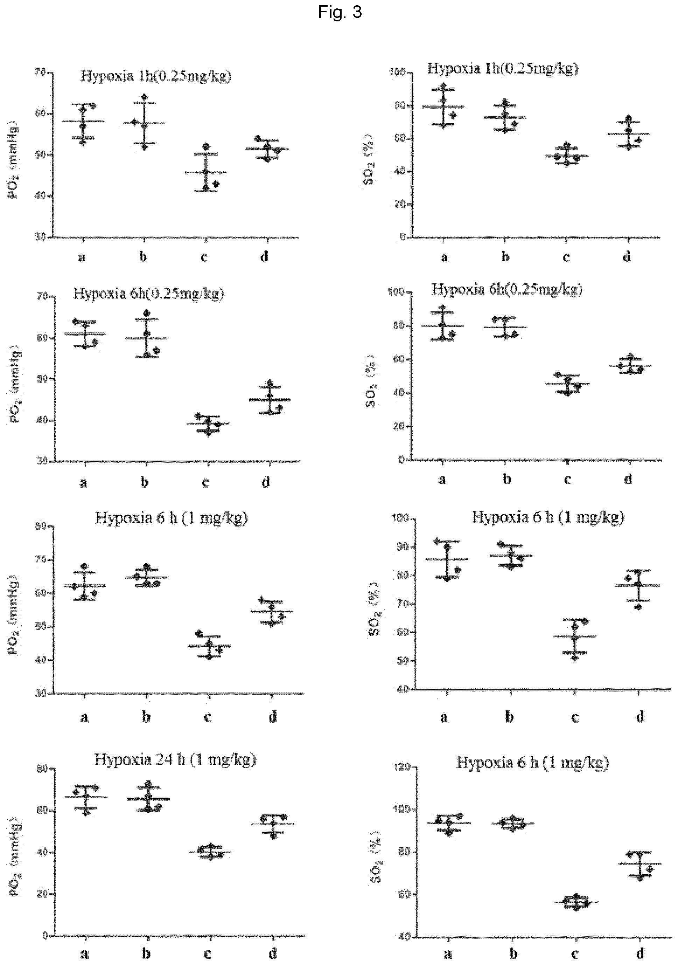

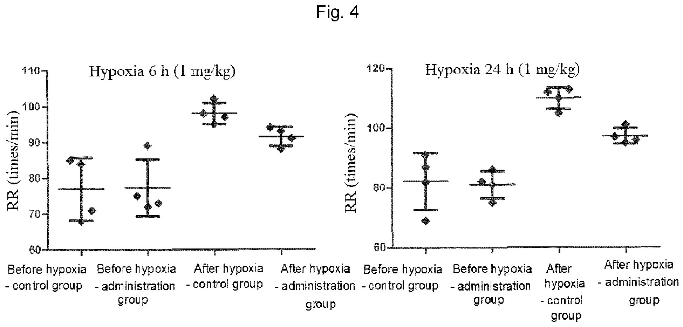

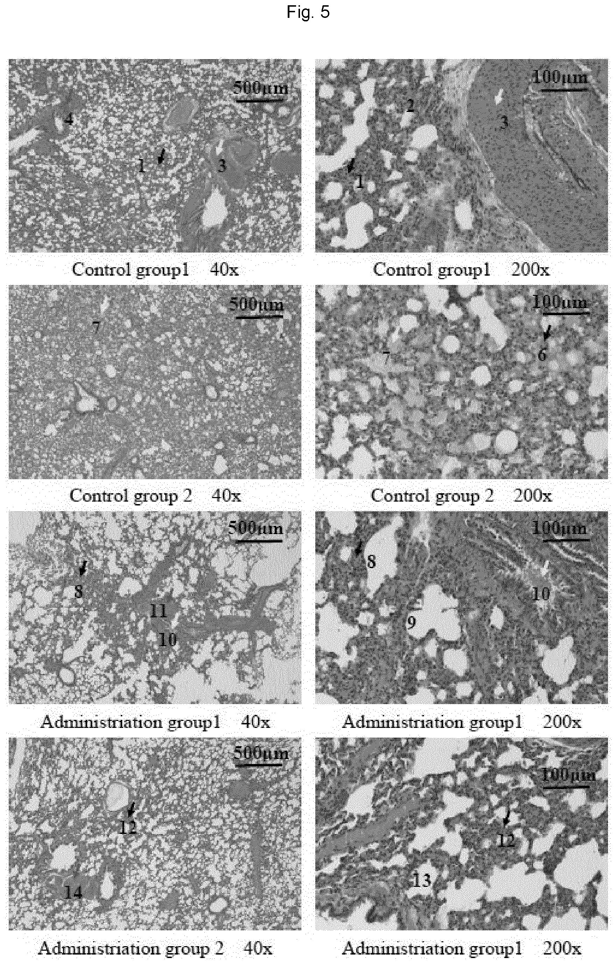

1. The blood gas analysis was shown in Fig. 3 (namely the effects of Compound 2 intravenously injected on rat blood oxygen partial pressure (PO₂) and blood oxygen saturation (SO₂) before 24 h of the hypoxia treatment and after 1 h, 6 h, and 24 h of the hypoxia treatment):
   1) Before 24 h of the hypoxia treatment, there was no significant difference in blood gas analysis results among each group of animals.
   2) After 1 h of the hypoxia treatment, the blood oxygen partial pressure of the 0.25 mg/kg administration group was higher than that of the control group, but the difference was not significant (p=0.059). The blood oxygen saturation of the administration group was higher than that of the control group, and the difference was significant (p=0.023).
   3) After 6 h of the hypoxia treatment, the blood oxygen partial pressure (p=0.018) and blood oxygen saturation (p=0.015) of the 0.25 mg/kg administration group were higher than those of the control group, and the difference was significant.
   4) After 6 h of the hypoxia treatment, the blood oxygen partial pressure of the 1 mg/kg administration group was higher than that of the control group, and the difference was significant (p=0.003). The blood oxygen saturation of the administration group was higher than that of the control group, and the difference was significant (p=0.0012).
   5) After 24 h of the hypoxia treatment, the blood oxygen partial pressure (p=0.0012) and blood oxygen saturation (p=0.001) of the 1 mg/kg administration group were higher than those of the control group, and the difference was significant.
2. The respiratory rate was shown in Fig. 4 (namely the effects of Compound 2 intravenously injected on the RR of SD rats before 24 h of the hypoxia treatment and after 6 h and 24 h of the hypoxia treatment):
   1) Before 24 h of the hypoxia treatment, there was no significant difference in the respiratory rate among each group of the animals.
   2) After 6 h or 24 h of the hypoxia treatment, the respiratory rate of the administration group was lower than that of the control group, and the difference was significant (p<0.05).
3. Pathological analysis of lung tissue paraffin sections was shown in Fig. 5 (namely the effects of Compound 2 intravenously injected on lung tissue injuries of the SD rats after 24 h of the hypoxia treatment):

From Control group 1, it might be seen that there was a large area of thickening of alveolar walls in the lung tissues (black arrow 1), accompanied by inflammatory cell infiltration (red arrow 2); a small amount of vascular muscularization, smooth muscle cell proliferation, thickening of vessel walls, and narrowing of lumens (yellow arrow 3); and occasionally, small focal infiltration of inflammatory cells around bronchia (blue arrow 4).

From Control group 2, it might be seen that there was a small amount of inflammatory cell infiltration on the alveolar walls in the lung tissues (black arrow 6); and extensive pulmonary edema, with homogeneous eosinophilic tissue fluid exudation visible in alveolar cavities (yellow arrow 7).

From Administration group 1, it might be seen that there was a moderate area of thickening of the alveolar walls in the lung tissues (black arrow 8), accompanied by a small amount of inflammatory cell infiltration (red arrow 9); a small amount of eosinophilic tissue fluid exudation visible in the lumens of the bronchia (yellow arrow 10); and a small amount of vascular muscularization, smooth muscle cell proliferation, thickening of the vessel walls, and narrowing of the lumens (blue arrow 11).

From Administration group 2, it might be seen that there was a large area of mild thickening of the alveolar walls in the lung tissues (black arrow 12), accompanied by a small amount of inflammatory cell infiltration (red arrow 13); and a small amount of vascular muscularization, smooth muscle cell proliferation, thickening of the vessel walls, and narrowing of the lumens (blue arrow 14).

In conclusion, compared with the control group, the administration group was able to alleviate the tissue injuries caused by hypoxia (anoxia), such as pulmonary edema, thickening of the alveolar walls, infiltration of inflammatory cells, infiltration of neutrophils, vascular muscularization, smooth muscle cell proliferation, thickening of the vessel walls, and narrowing of the lumens.

From the above data, it might be seen that 0.25 mg/kg and 1 mg/kg of Compound 2 intravenously injected once might increase the blood oxygen partial pressure and blood oxygen saturation of the SD rats after 1 h, 6 h, and 24 h of the hypoxia treatment; 1 mg/kg of Compound 2 intravenously injected once might significantly reduce the respiratory rate of the SD rats after 6 h and 24 h of the hypoxia treatment; and 1 mg/kg of Compound 2 intravenously injected once might significantly reduce the injuries to the lung tissues of the SD rats after 24 h of the hypoxia treatment. Accordingly, it was indicated that Compound 2 injected might effectively resist hypoxic/anoxic injuries by increasing the blood oxygen carrying capacity.

### Embodiment 3

### (S)-2,6-diamino-N-(3',5-diallyl-2,4'-dihydroxy-[1,1'-biphenyl]-3-yl)hexanamide dihydrochloride (Compound 2):

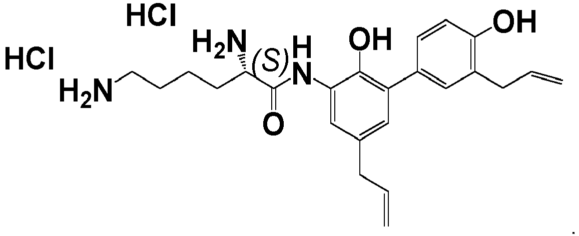

### I. Experimental method

1. Preparation of test substance: an appropriate amount of Compound 2 was weighed, and an appropriate amount of sodium chloride injection (0.9%) was added, to prepare solution with a required concentration (40 mg/kg).
2. Animal model: SPF-grade SD male rats, 220-240 g of the weight, and hypoxia (the oxygen content was 11%) treatment.
3. Experimental grouping: a hypoxia treatment control group, there were 4 rats in each group; and a hypoxia treatment administration group, there were 4 rats in each group.
4. Administration mode: single gavage administration of each rat in the hypoxic treatment group prior to hypoxic treatment.
5. Blood gas analysis: blood (approximately 0.5 mL) was respectively taken from femoral arteries of each group before and after 24 h of the hypoxia treatment for blood gas analysis, and it referred to Table 3 for details.

**Table 3**

| Group | Dose concentration (mg/kg) | Blood gas analysis time |
|---|---|---|
| Hypoxia treatment control group | - | Blood is taken once before 24 h of the hypoxia treatment; and blood is taken once after 24 h of the hypoxia treatment |
| Hypoxia treatment administration group | 40 | Blood is taken once before 24 h of the hypoxia treatment; and blood is taken once after 24 h of the hypoxia treatment |

6. Respiratory rate: the rat respiratory rate was evaluated during blood collection.
7. Data statistics: GraphPad was used for data analysis, and results were expressed as Mean±SD. Single factor analysis of variance - T-test was used for data analysis, and P<0.05 represented that it had a statistical difference.

### II. Experimental result

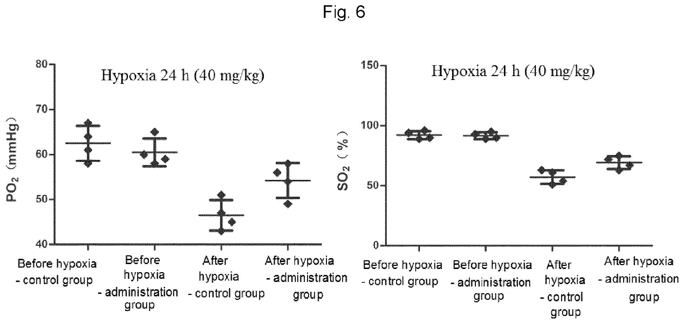

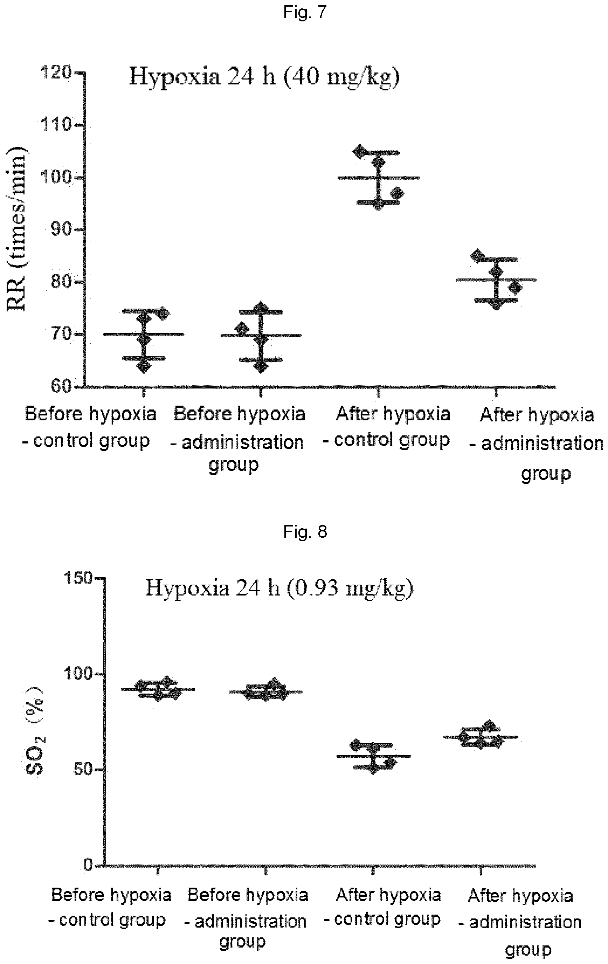

1. The blood gas analysis was shown in Fig. 6 (the effects of Compound 2 intragastrically administered on the blood oxygen partial pressure (PO₂) and blood oxygen saturation (SO₂) before and after 24 h of the hypoxia treatment):
   1) Before 24 h of the hypoxia treatment, there was no significant difference in the blood gas analysis results among each group of the animals.
   2) After 24 h of the hypoxia treatment, the blood oxygen partial pressure (p=0.036) and blood oxygen saturation (p=0.016) of the 40 mg/kg administration group were higher than those of the control group, and the difference was significant.
2. The respiratory rate was shown in Fig. 7 (the effects of Compound 2 intragastrically administered on the RR before and after 24 h of the hypoxia treatment):
   1) Before 24 h of the hypoxia treatment, there was no significant difference in the respiratory rate among each group of the animals.
   2) After 24 h of the hypoxia treatment, the respiratory rate of the administration group was lower than that of the control group, and the difference was significant (p<0.05).

From the above data, it might be seen that 40 mg/kg of Compound 2 intragastrically administered once might significantly increase the blood oxygen partial pressure and blood oxygen saturation of the SD rats after 24 h of the hypoxia treatment, and reduce the respiratory rate. Accordingly, it was indicated that Compound 2 intragastrically administered might effectively resist hypoxic/anoxic injuries by increasing the blood oxygen carrying capacity.

### Embodiment 4

### (S)-2-amino-N-(3',5-diallyl-2,4'-dihydroxy-[1,1'-biphenyl]-3-yl)-4-(methylthio)butanamid e hydrochloride (Compound 3):

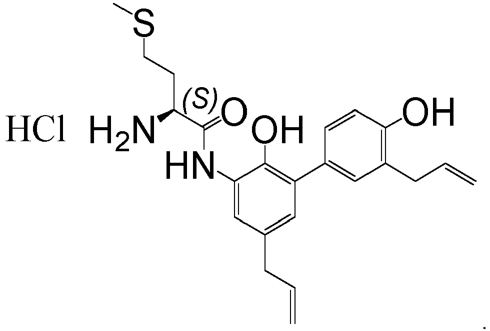

### I. Experimental method

1. Preparation of test substance: an appropriate amount of Compound 3 was weighed, and an appropriate amount of sodium chloride injection (0.9%) was added, to prepare solution with a required concentration (0.93 mg/kg).
2. Animal model: SPF-grade SD male rats, 220-240 g of the weight, and hypoxia (the oxygen content was 11%) treatment.
3. Experimental grouping: a hypoxia treatment control group, there were 4 rats in each group; and a hypoxia treatment administration group, there were 4 rats in each group.
4. Administration mode: before the hypoxia treatment, a single intravenous injection was administered to each rat in the hypoxia treatment administration group.
5. Blood gas analysis: blood (approximately 0.5 mL) was respectively taken from femoral arteries of each group before and after 24 h of the hypoxia treatment for blood gas analysis, and it referred to Table 4 for details.

**Table 4**

| Group | Dose concentration (mg/kg) | Blood gas analysis time |
|---|---|---|
| Hypoxia treatment control group | - | Blood is taken once before 24 h of the hypoxia treatment; and blood is taken once after 24 h of the hypoxia treatment |
| Hypoxia treatment administration group | 0.93 | Blood is taken once before 24 h of the hypoxia treatment; and blood is taken once after 24 h of the hypoxia treatment |

6. Data statistics: GraphPad was used for data analysis, and results were expressed as Mean±SD. Single factor analysis of variance - T-test was used for data analysis, and P<0.05 represented that it had a statistical difference.

### II. Experimental result

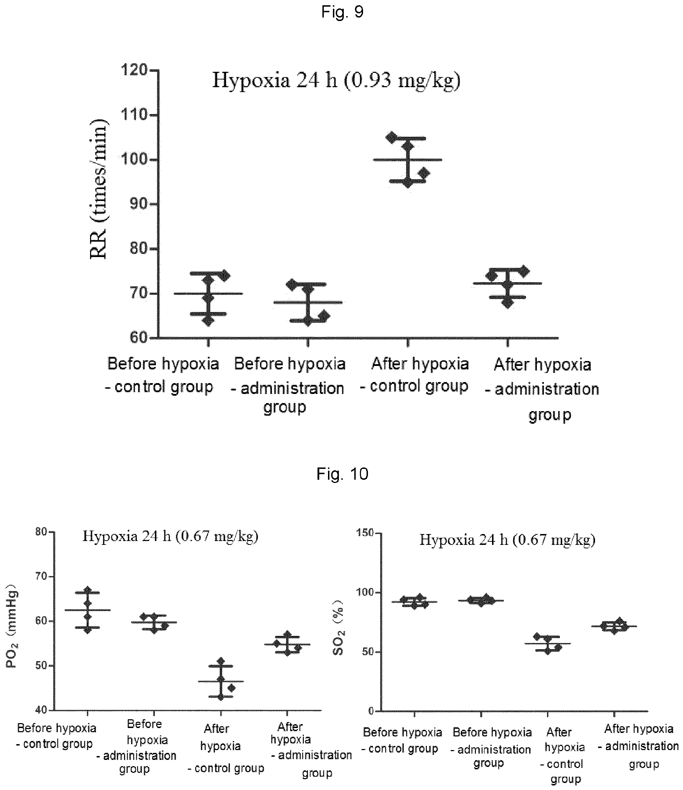

1. The blood gas analysis was shown in Fig. 8 (the effects of Compound 3 on the blood oxygen saturation (SO₂) of the SD rats before and after 24 h of the hypoxia treatment):
   1) Before 24 h of the hypoxia treatment, there was no significant difference in the blood gas analysis results among each group of the animals.
   2) After 24 h of the hypoxia treatment, the blood oxygen saturation (p=0.06) of the 0.93 mg/kg administration group was higher than that of the control group.
2. The respiratory rate was shown in Fig. 9 (the effects of Compound 3 on the rat RR before and after 24 h of the hypoxia treatment):
   1) Before 24 h of the hypoxia treatment, there was no significant difference in the respiratory rate among each group of the animals.
   2) After 24 h of the hypoxia treatment, the respiratory rate of the administration group was lower than that of the control group, and the difference was significant (p<0.05).
From the above data, it might be seen that 0.93 mg/kg of Compound 3 intravenously injected once might increase the blood oxygen saturation of the SD rats after 24 h of the hypoxia treatment, significantly reduce the respiratory rate, and thus play an anti-hypoxic/anoxic injury role.

### Embodiment 5

### N-(3',5-diallyl-2,4'-dihydroxy-[1,1'-biphenyl]-3-yl)acetamide (Compound 4):

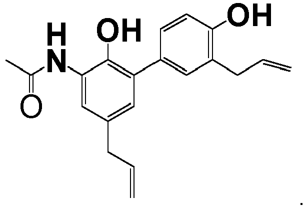

### I. Experimental method

1. Preparation of test substance: an appropriate amount of Compound 4 was weighed, and an appropriate amount of solvent 2% DMSO + 2% Tween80 + physiological saline was added, to prepare solution with a required concentration (0.67 mg/kg).
2. Animal model: SPF-grade SD male rats, 220-240 g of the weight, and hypoxia (the oxygen content was 11%) treatment.
3. Experimental grouping: a hypoxia treatment control group, there were 4 rats in each group; and a hypoxia treatment administration group, there were 4 rats in each group.
4. Administration mode: before the hypoxia treatment, a single intravenous injection was administered to each rat in the hypoxia treatment administration group.
5. Blood gas analysis: blood (approximately 0.5 mL) was respectively taken from femoral arteries of each group before and after 24 h of the hypoxia treatment for blood gas analysis, and it referred to Table 5 for details.

**Table 5**

| Group | Dose concentration (mg/kg) | Blood gas analysis time |
|---|---|---|
| Hypoxia treatment control group | - | Blood is taken once before 24 h of the hypoxia treatment; and blood is taken once after 24 h of the hypoxia treatment |
| Hypoxia treatment administration group | 0.67 | Blood is taken once before 24 h of the hypoxia treatment; and blood is taken once after 24 h of the hypoxia treatment |

6. Respiratory rate: the rat respiratory rate was evaluated during blood collection.
7. Data statistics: GraphPad was used for data analysis, and results were expressed as Mean±SD. Single factor analysis of variance - T-test was used for data analysis, and P<0.05 represented that it had a statistical difference.

### II. Experimental result

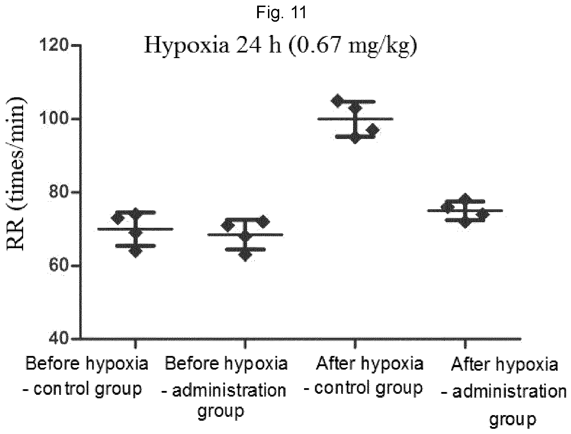

1. The blood gas analysis was shown in Fig. 10 (the effects of Compound 4 on the blood oxygen partial pressure (PO₂) and blood oxygen saturation (SO₂) of the SD rats before and after 24 h of the hypoxia treatment):
   1) Before 24 h of the hypoxia treatment, there was no significant difference in the blood gas analysis results among each group of animals.
   2) After 24 h of the hypoxia treatment, the blood oxygen partial pressure (p=0.023) and blood oxygen saturation (p=0.0023) of the 0.67 mg/kg administration group were higher than those of the control group.
2. The respiratory rate was shown in Fig. 11 (the effects of Compound 4 on the rat RR before and after 24 h of the hypoxia treatment):
   1) Before 24 h of the hypoxia treatment, there was no significant difference in the respiratory rate among each group of the animals.
   2) After 24 h of the hypoxia treatment, the respiratory rate of the administration group was lower than that of the control group, and the difference was significant (p<0.05).

0.67 mg/kg of Compound 4 injected once might significantly increase the blood oxygen partial pressure and blood oxygen saturation of the SD rats after 24 h of the hypoxia treatment, significantly reduce the respiratory rate, and thus play an anti-hypoxic/anoxic injury role.

In conclusion, it may be concluded that the use of the magnolol and/or honokiol aromatic ring amino-substituted derivative of the present disclosure in the preparation of the anti-hypoxic/anoxic injury drug may significantly exert the anti-hypoxic/anoxic injury effect.

From the above description, it may be seen that the above embodiments of the present disclosure achieve the following technical effects:

The derivative with the structure shown in the above general formula I may be used to prepare the anti-hypoxic/anoxic injury drug, rapidly and effectively improve the oxygen carrying capacity of red blood cells, provide a new drug choice for prevention and treatment of diseases (such as altitude sickness) caused by external hypoxia and breathing and/or ventilation dysfunction diseases caused by various self-causes (such as central nervous system diseases, bronchial and pulmonary lesions), and solve problems of indirect onset and slow onset and the like of existing anti-hypoxic/anoxic drugs.

The above are only preferred embodiments of the present disclosure and are not intended to limit the present disclosure. For those skilled in the art, the present disclosure may have various modifications and variations. Any modifications, equivalent replacements, improvements and the like made within the spirit and principles of the present disclosure shall be included within the scope of protection of the present disclosure.

## Claims

1. An anti-hypoxic/anoxic injury use of a magnolol and/or honokiol aromatic ring amino-substituted derivative, wherein the magnolol and/or honokiol aromatic ring amino-substituted derivative is a compound shown in general formula I or a salt thereof: 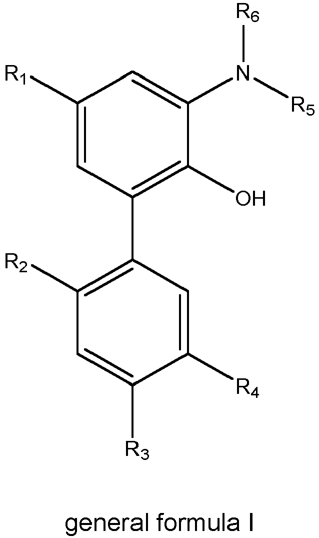 in general formula I, R₁ and R₄ are each independently selected from a C₁-C₈ hydrocarbyl, R₂ and R₃ are each independently selected from hydrogen or hydroxyl, and R₂ and R₃ are not both the hydrogen or hydroxyl; R₅ is selected from any one of H, a C₁-C₁₂ acyl, a remaining acyl moiety of a single amino acid after condensation of its carboxyl, or a remaining acyl moiety of a polypeptide after condensation of its carboxyl; and R₆ is selected from any one of hydrogen or C₁-C₈ hydrocarbyl.

2. The anti-hypoxic/anoxic injury use according to claim 1, wherein the C₁-C₈ hydrocarbyls in the R₁, R₄, and R₆ are each independently selected from any one of a C₁-C₈ alkyl or a C₁-C₈ alkenyl, and preferably the C₁-C₈ alkyl is selected from any one of methyl, ethyl, propyl, butyl, pentyl, hexyl, heptyl, or octyl.

3. The anti-hypoxic/anoxic injury use according to claim 2, wherein the C₁-C₈ alkenyl is selected from any one of ethenyl, propenyl, allyl, butan-1-enyl, butan-2-enyl, butan-3-enyl, pentan-1-enyl, pentan-2-enyl, pentan-3-enyl, pentan-4-enyl, hex-1-enyl, hex-2-enyl, hex-3-enyl, hex-4-enyl, hex-5-enyl, hept-1-enyl, hept-2-enyl, hept-3-enyl, hept-4-enyl, hept-5-enyl, hept-6-enyl, oct-1-enyl, oct-2-enyl, oct-3-enyl, oct-4-enyl, oct-5-enyl, oct-6-enyl, or oct-7-enyl.

4. The anti-hypoxic/anoxic injury use according to any one of claims 1 to 3, wherein the C₁-C₁₂ acyl is selected from any one of formyl, acetyl, propionyl, butyryl, valeryl, hexanoyl, heptanoyl, or octanoyl.

5. The anti-hypoxic/anoxic injury use according to any one of claims 1 to 4, wherein the single amino acid is selected from any one of lysine, methionine, tryptophan, valine, alanine, phenylalanine, leucine, isoleucine, 6-hydroxyleucine, glycine, histidine, arginine, proline, glutamic acid, aspartic acid, serine, threonine, tyrosine, cystine or cysteine.

6. The anti-hypoxic/anoxic injury use according to any one of claims 1 to 5, wherein the polypeptide is a peptide formed by a plurality of single amino acids, and preferably the molecular weight of the polypeptide is ≤2500 Da.

7. The anti-hypoxic/anoxic injury use according to any one of claims 1 to 6, wherein when the magnolol and/or honokiol aromatic ring amino-substituted derivative is the salt of the compound shown in general formula I, one or more of salifiable amino groups carried by R₅ are in salt form, and an acid used for salt formation is a pharmaceutically acceptable acid such as hydrochloric acid, oxalic acid, or fumaric acid.

8. The anti-hypoxic/anoxic injury use according to any one of claims 1 to 7, wherein the magnolol and/or honokiol aromatic ring amino-substituted derivative is any one or more of the following compounds: 3',5-diallyl-3-amino-[1,1'-biphenyl]-2,4'-diol and hydrochloride thereof, N-(3',5-diallyl-2,4'-dihydroxy-[1,1'-biphenyl]-3-yl)acetamide, (S)-2,6-diamino-N-(3',5-diallyl-2,4'-dihydroxy-[1,1'-biphenyl]-3-yl)hexanamide and hydrochloride thereof, (S)-2-amino-N-(3',5-diallyl-2,4'-dihydroxy-[1,1'-biphenyl]-3-yl)-3-phenylpropanamide and hydrochloride thereof, (R)-2,6-diamino-N-(3',5-diallyl-2,4'-dihydroxy-[1,1'-biphenyl]-3-yl)hexanamide and hydrochloride thereof, (S)-2-amino-N-(3',5-diallyl-2,4'-dihydroxy-[1,1'-biphenyl]-3-yl)-6-hydroxyhexanamide and hydrochloride thereof, (S)-2-amino-N-(3',5-diallyl-2,4'-dihydroxy-[1,1'-biphenyl]-3-yl)-4-(methylthio)butanamide and hydrochloride thereof, (S)-2-amino-N-(3',5-diallyl-2,4'-dihydroxy-[1,1'-biphenyl]-3-yl)-3-methylbutanamide and hydrochloride thereof, and (S)-2,6-diamino-N-(2,4'-dihydroxy-3',5-dipropyl-[1,1'-biphenyl]-3-yl)hexanamide and hydrochloride thereof.

9. The anti-hypoxic/anoxic injury use according to claim 1, wherein the administration mode of the anti-hypoxic/anoxic injury drug is injection, oral administration, implantation, or direct filling of lesion sites.

10. An anti-hypoxic/anoxic injury pharmaceutical composition, wherein the anti-hypoxic/anoxic injury pharmaceutical composition comprises a magnolol and/or honokiol aromatic ring amino-substituted derivative or a salt thereof, as well as a pharmaceutically acceptable carrier; and wherein the magnolol and/or honokiol aromatic ring amino-substituted derivative is the magnolol and/or honokiol aromatic ring amino-substituted derivative according to claim 1.
